# EUROPÄISCHE PATENTSCHRIFT

(11) **EP 2 692 855 B1**
(45) Veröffentlichungstag und Bekanntmachung des Hinweises auf die Patenterteilung: **01.05.2019**
(21) Anmeldenummer: 13176891.3
(22) Anmeldetag: 17.07.2013
(51) Int. Cl.: C12M 3/00, C12M 1/24, C12M 1/22, C12M 3/06, C12M 1/00

(54) **Oberflächenstrukturierung für zellbiologische und/oder medizinische Anwendungen**
Surface structuring for cytological and/or medical applications
Structuration de surface pour des applications médicales et/ou de biologie cellulaire

(30) Priorität: 03.08.2012 DE 102012213787
(43) Veröffentlichungstag der Anmeldung: 05.02.2014
(73) Patentinhaber: Robert Bosch GmbH, 70442 Stuttgart (DE)
(72) Erfinder: Dudziak, Sonja, 74321 Bietigheim-Bissingen (DE); Hoerlein, Rainer Heinrich, 70376 Stuttgart (DE)

(56) Entgegenhaltungen:
- WO-A1-01/78891
- WO-A1-99/36276
- WO-A1-2005/093470
- WO-A1-2009/125284
- WO-A2-00/73856
- WO-A2-2006/102347
- WO-A2-2011/111069
- DE-A1- 4 018 804
- DE-A1-102007 006 634
- DE-A1-102008 040 782
- DE-A1-102010 034 085
- US-A- 6 015 759
- US-A1- 2009 093 879
- US-A1- 2009 093 881

## Beschreibung

Die vorliegende Erfindung betrifft eine Vorrichtung mit Oberflächenstrukturierung für zellbiologische und/oder medizinische Anwendungen gemäß Anspruch 1. Stand

### der Technik

Für viele biologische und/oder medizinische Anwendungen werden künstliche Umgebungen geschaffen, um eine Ansiedlung und Proliferation von eukaryotischen Zellen oder Bakterien zu ermöglichen. So sind beispielsweise eine Vielzahl unterschiedlicher Zellkulturgefäße bekannt, die für eine Kultur verschiedenartiger Zellen oder Bakterien geeignet sind, beispielsweise Kulturschalen oder Flaschen. Dabei ist bekannt, dass die Ansiedlung und die Proliferation von vielen Zelltypen oder Zellsorten entscheidend auch von Oberflächenbeschaffenheiten abhängig ist. Dies gilt insbesondere für Zellen, die an einem Substrat adhärieren, beispielsweise Makrophagen oder Endothelzellen.

Die DE102007006634 offenbart ein Verfahren und eine Vorrichtung zur PTFE-Oberflächenmodifizierung und beschreibt, dass bei geeignet hoher Pulsenergiedichte eine Bestrahlung mit vergleichsweise leicht zu erzeugenden Nanosekunden-Pulsen im UV-Wellenlängenbereich über 180 nm eine Oberflächenmodifizierung erreicht wurde, die im Hinblick auf das Anwachsen von biologischen Zellen eine Verbesserung der Hafteigenschaften zeigte.

Auch medizinische Implantate können in Hinblick auf eine Ansiedlung und Proliferation von Zellen, insbesondere von körpereigenen Zellen, angepasst und optimiert sein. Es ist beispielsweise bekannt, Hüftendoprothesen schaftseitig mit einer Pulverbeschichtung zu versehen, um die bevorzugte Ansiedlung von Knochenvorläuferzellen zu ermöglichen. Auch die Oberflächen von kardiovaskulären Gefäßstützen (Stents) können so beschichtet werden, dass Endothelzellen bevorzugt anwachsen, während eine Thrombozytenanlagerung oder ein Muskelzellwachstum auf den inneren Oberflächen des Stents vermieden wird.

Die bisherigen Ansätze zur Optimierung von Oberflächen für verschiedene zellbiologische und/oder medizinische Anwendungen führen oftmals nicht zu befriedigenden Ergebnissen. Demgegenüber liegt der Erfindung die Aufgabe zugrunde, eine Vorrichtung mit Oberflächenstrukturierung für zellbiologische und/oder medizinische Anwendungen bereitzustellen. Derart herstellbare Vorrichtungen sollen verbesserte Oberflächenstrukturen aufweisen, um beispielsweise die Anheftung und Proliferation von Zellen oder Bakterien zu fördern oder gegebenenfalls zu hemmen oder um durch Einlagerung von bestimmten Substanzen die Anheftung und/oder das Wachstum von Zellen oder Bakterien zu beeinflussen.

### Offenbarung der Erfindung

### Vorteile der Erfindung

Diese Aufgabe wird durch ein Vorrichtungen für zellbiologische und/oder medizinische Anwendungen gemäß Anspruch 1 gelöst. Durch die in den abhängigen Ansprüchen aufgeführten Maßnahmen sind vorteilhafte Weiterbildungen und Verbesserungen der erfindungsgemäßen Vorrichtung möglich.

Die Oberflächenstrukturierung von Vorrichtungen für zellbiologische und/oder medizinische Anwendungen beruht darauf, dass auf wenigstens einer Oberfläche zumindest teilweise mittels elektromagnetischer Strahlung eine Oberflächenstruktur erzeugt wird, die eine von einer Nanostruktur überlagerte Mikrostruktur aufweist. Die von der Nanostruktur überlagerte Mikrostruktur ist so zu verstehen, dass die Oberflächenstruktur auch im Wesentlichen nur von einer Nanostruktur oder nur von einer Mikrostruktur geprägt sein kann. Die erfindungsgemäße Oberflächenstrukturierung erlaubt die Erzeugung verschiedener Oberflächenbereiche mit lokal angepassten Eigenschaften beispielsweise für die Zell- und/oder Bakterienkultur. Die Eigenschaften werden maßgeblich durch die Morphologie der Oberflächen-Nanostruktur und/oder -Mikrostruktur hervorgerufen, die durch entsprechende Parameterwahl der elektromagnetischen Strahlung beispielsweise in einem Laserprozess eingestellt werden kann.

Eine vergleichbare Oberflächenstrukturierung ist bereits aus der DE 10 2008 040 782 A1 bekannt. Hier wird eine Oberflächenstrukturierung beschrieben, um einen gut haftenden Bauteilverbund zweier Bauteile insbesondere für Kraftfahrzeuganwendungen bereitstellen zu können. Die Oberfläche eines ersten Bauteils insbesondere aus Metall wird mit einer Mikrostruktur versehen, die von einer Nanostruktur überlagert ist. Auf dieses erste Bauteil wird ein zweites Bauteil aus thermoplastischem oder duroplastischem Kunststoff aufgebracht. Hierbei bewirkt die Oberflächenstrukturierung eine besonders gute Haftung der Bauteile aneinander. Diese durch elektromagnetische Strahlung erzeugte Oberflächenstruktur wird gemäß der vorliegenden Erfindung genutzt, um Oberflächen für zellbiologische und/oder medizinische Zwecke anzupassen und zu optimieren. Insbesondere kann durch eine derartige Oberflächenstrukturierung eine Ansiedlung und eine Proliferation verschiedener Zelltypen und Bakterien gezielt beeinflusst werden. Weiterhin kann durch eine derartige Oberflächenstrukturierung beispielsweise auch ein Bakterien- oder Zellwachstum gehemmt werden. Somit kann eine entsprechend behandelte Oberfläche mit lokal angepassten Eigenschaften im Hinblick auf zellbiologische und/oder medizinische Zwecke versehen werden. Über die Parameter der elektromagnetischen Strahlung wird dabei die Morphologie der Mikro- und/oder der Nanostruktur kontrolliert. Durch eine Änderung der Prozessparameter während des Strukturierungsprozesses ist es möglich, die Oberflächen bedarfsangepasst zu strukturieren, um gezielt das Anwachsen und die Proliferation von Bakterien, Zellen oder anderem, beispielsweise Geweben, zu fördern oder zu hemmen. Weiterhin ist es möglich, lokale Depots für verschiedene Substanzen, beispielsweise Nährmedien, Botenstoffe, Medikamente oder andere Wirkstoffe zu schaffen, die für eine Zell- oder Gewebekultur oder auch für andere Zwecke genutzt werden können.

Die Oberflächenstrukturierung wird Hilfe eines Lasers, insbesondere mit einem Ultrakurzpulslaser, erzeugt. Hierbei wird die Oberflächenstruktur vorzugsweise unter einem Prozessmedium, insbesondere unter einer Prozessgas-Atmosphäre, vorzugsweise einer Inertgas-Atmosphäre, erzeugt. Durch die Prozessgas-Atmosphäre kann die Effizienz der Oberflächenstrukturierung weiter erhöht werden. Weiterhin kann die Prozessgas-Atmosphäre für eine chemische Veränderung der Oberflächen genutzt werden, sodass beispielsweise eine Passivierung erzeugt wird. Als Prozessgas kann beispielsweise Helium oder Argon eingesetzt werden, das, je nach verwendetem Material für die Vorrichtung, beispielsweise die Bildung einer Oxidschicht verhindern kann.

Als Material für die Vorrichtungen, deren Oberflächen strukturiert werden, können insbesondere Kunststoffe eingesetzt werden. Es können beispielsweise Schalen oder Flaschen aus Kunststoff, die üblicherweise für eine Zellkultur eingesetzt werden, erfindungsgemäß bearbeitet werden, um optimierte Oberflächen für die entsprechenden zellbiologischen und/oder medizinischen Anwendungen bereitstellen zu können. Weiterhin können auch metallische Werkstoffe für die Herstellung der erfindungsgemäß zu bearbeitenden Vorrichtungen eingesetzt werden, beispielsweise für Gefäße oder Implantate. So können beispielsweise Magnesium, Edelstahl oder Titan für medizinische Implantate eingesetzt werden, die erfindungsgemäß bearbeitet werden. Weiterhin ist es möglich, beispielsweise keramische Werkstoffe oder Glas einzusetzen.

Bevorzugterweise liegt die Strahlungswellenlänge der elektromagnetischen Strahlung in einem Wertebereich zwischen etwa 10 nm und etwa 11 µm. Besonders bevorzugt ist eine Wellenlänge zwischen etwa 200 nm und etwa 1500 nm. Die Strahlungspulsdauer kann vorzugsweise in einem Wertebereich zwischen etwa 10 fs und etwa 10 µs liegen, insbesondere zwischen etwa 100 fs und etwa 100 ps. Durch die Wahl der entsprechenden Strahlungsparameter kann die gewünschte Oberflächenstruktur, die eine von einer Nanostruktur überlagerte Mikrostruktur aufweist, erzeugt werden. Hierbei ist die Morphologie der Mikro- und/oder der Nanostruktur maßgeblich von der Wahl der Prozessparameter abhängig. Sie kann somit gezielt beeinflusst werden, um die erfindungsgemäß beabsichtigten Eigenschaften einzustellen, also insbesondere die Förderung der Ansiedlung und/oder Proliferation bestimmter Zelltypen oder Zellsorten oder bestimmter Bakterien, Geweben oder ähnlichem, oder die Hemmung einer entsprechenden Ansiedlung oder die Einlagerung verschiedener Wirkstoffe, Nährmedien, Botenstoffe oder ähnlichem. Die Mikrostruktur der erfindungsgemäß erzeugten Oberfläche weist vorzugsweise Mikrostrukturelemente auf, die einen Durchmesser in einem Größenbereich zwischen etwa 1 µm bis etwa 999 µm aufweisen. Die Nanostrukturelemente der Nanostruktur der erfindungsgemäß erzeugten Oberfläche weisen vorzugsweise einen Durchmesser in einem Größenbereich zwischen etwa 1 nm und etwa 999 nm auf. Durch die Anpassung der Prozessparameter können Mikrostrukturelemente und Nanostrukturelemente insbesondere aus diesen Größenbereichen erzeugt werden, die für die jeweiligen Anwendungen besonders geeignet sind. So ist es beispielsweise bekannt, dass Knochenzellen Strukturelemente mit Abmessungen von cira 100 - 300 µm bevorzugen, wobei eine Nanostrukturierung der Oberfläche die Adhäsion der Zellen begünstigt.

Die Erfindung umfasst Vorrichtungen für zellbiologische und/oder medizinische Anwendungen, die Oberflächen aufweisen, die zumindest teilweise eine durch elektromagnetische Strahlung erzeugte Oberflächenstruktur zeigen, wobei die Oberflächenstruktur durch eine Mikrostruktur gekennzeichnet ist, die von einer Nanostruktur überlagert ist. Die Oberflächenstruktur weist insbesondere in ihrer Mikrostruktur Mikrostrukturelemente mit einem Durchmesser aus einem Größenbereich zwischen etwa 1 µm bis etwa 999 µm und in ihrer Nanostruktur Nanostrukturelemente mit einem Durchmesser aus einem Größenbereich zwischen etwa 1 nm und etwa 999 nm auf.

Durch eine Anpassung der Oberflächenstrukturelemente wird erfindungsgemäß die Oberflächenstruktur für das Wachstum oder auch die Hemmung vorgebbarer Zellsorten, Zelllinien und/oder Gewebe und/oder für die Einlagerung von Wirkstoffen oder anderen Substanzen, beispielsweise Nährmedien, optimiert sein. Entsprechend geeignete Oberflächenstrukturen werden durch die Einstellung geeigneter Prozessparameter bei der Erzeugung der Oberflächenstrukturen eingestellt. So kann beispielsweise die Strahlungswellenlänge der elektromagnetischen Strahlung und/oder die Strahlungspulsdauer eines eingesetzten Lasers zur Erzeugung der gewünschten Strukturelemente entsprechend eingestellt werden. Durch die Wahl eines bestimmten Prozessmediums kann die Oberflächenstrukturierung weiter beeinflusst werden.

Die erfindungsgemäße Vorrichtung weist eine Oberfläche oder Oberflächen auf die oberflächenstrukturiert ist (sind), zwei oder mehr Bereiche mit unterschiedlichen Oberflächenstrukturen. Es werden Oberflächenbereiche mit sehr kleinen Abmessungen, beispielsweise kleiner als 10.000 µm² mit unterschiedlichen Eigenschaften auch in direkter Nachbarschaft erhalten. Die Geometrie der Oberflächenbereiche ist dabei maßgeblich durch die Geometrie der Oberfläche selbst bzw. der Vorrichtung selbst limitiert. Alle Bereiche, die für die elektromagnetische Strahlung, insbesondere einen Laserstrahl, zugänglich sind, können prinzipiell strukturiert werden. Dabei können die Laserparameter bei jedem Laserpuls variiert werden, sodass die zugänglichen Oberflächenbereiche prinzipiell mit beliebigen Mustern strukturiert werden können. Eine solche Geometriefreiheit ist bei herkömmlichen Ätz- oder Beschichtungsverfahren oder beispielsweise auch bei einer Strukturierung durch Sandstrahlprozesse oder ähnliches nicht möglich. Durch die erfindungsgemäße Oberflächenstrukturierung können daher die Oberflächen in einzelnen Bereichen für verschiedene Zwecke optimiert werden. So können beispielsweise in einer Zellkulturschale Strukturbereiche für verschiedene Zellsorten erzeugt werden, um eine Co-Kultur dieser Zellsorten zu ermöglichen bzw. zu optimieren. Weiterhin sind Strukturbereiche für die Einlagerung bestimmter Substanzen beispielsweise für Nährstoffe, Wachstumsstoffe oder ähnliches vorgesehen sein. Da die Oberflächenbereiche mit unterschiedlichen Eigenschaften durch eine Parametervariation in einem Strukturierungsprozess in dem gleichen Prozess erzeugt werden können.

Ein bevorzugtes Beispiel für eine erfindungsgemäße Vorrichtung ist ein Gefäß für die Zell- und/oder Bakterienkultur. Wie bereits beschrieben, kann durch eine Anpassung der Mikro- und Nanostrukturelemente der erfindungsgemäß hergestellten Oberflächenstrukturen eine Optimierung der Ansiedlung und Proliferation bestimmter Zellsorten oder Bakterien erreicht werden. Weiterhin können durch die erfindungsgemäß herstellbaren Oberflächen optimale Bedingungen beispielsweise auch für eine Gewebekultur geschaffen werden, sodass die erfindungsgemäße Vorrichtung auch ein Gefäß für eine Gewebekultur sein kann. Diese Gefäße können beispielsweise die Form üblicher Kulturschalen, Lochplatten oder Kulturflaschen aufweisen, die beispielsweise aus transparentem Kunststoff oder anderen üblichen Materialien hergestellt sind. Bei diesen Gefäßen sind vorzugsweise die Böden mit der erfindungsgemäßen Oberflächenstruktur versehen.

In besonders bevorzugter Weise ist die erfindungsgemäße Vorrichtung ein Gefäß für eine Co-Kultur verschiedener Zellen und/oder Bakterien und/oder Geweben. Durch die Möglichkeit der Erzeugung von verschiedenen Strukturbereichen auf einer Oberfläche können beispielsweise auf einer Zellkulturplatte optimale Bedingungen für verschiedene Zellsorten oder Zelltypen geschaffen werden. Für ein optimales Wachstum können weitere Strukturbereiche für eine Nährstoffeinlagerung oder für die Einlagerung von Wachstumsfaktoren oder anderem geschaffen werden, sodass eine solche Vorrichtung in besonderer Weise für die Co-Kultur geeignet ist. Derartige Gefäße können beispielsweise mit besonderem Vorteil für die Zell- oder Gewebekultur für regenerative medizinische Anwendungen genutzt werden.

In einer weiteren bevorzugten Ausführungsform der erfindungsgemäßen Vorrichtung handelt es sich bei der Vorrichtung um ein medizinisches Implantat, beispielsweise ein Lang- oder Kurzzeitimplantat. Oftmals ist es medizinisch vorteilhaft, Implantate so auszugestalten, dass eine Anheftung und ein Anwachsen bestimmter körpereigener Zellen gefördert oder gegebenenfalls verhindert werden soll. Die erfindungsgemäße Oberflächenstrukturierung erlaubt eine solche Anpassung eines Implantats in besonders vorteilhafter Weise.

In einer weiteren bevorzugten Ausgestaltung einer erfindungsgemäßen Vorrichtung handelt es sich bei der Vorrichtung um einen Mikrochip. Es sind bereits sogenannte Lap-on-a-Chip-Anwendungen bekannt, bei denen mikrofluidische Systeme in Chip-Form genutzt werden, um beispielsweise bestimmte biochemische Prozesse oder bestimmte Zellen in einer Probe nachweisen zu können. Hierfür sind bei einem solchen Chip bestimmte Bereiche für verschiedene Reagenzien, Zellen und bestimmte biochemische Reaktionen vorgesehen. Erfindungsgemäß können verschiedene Bereiche oder Kompartimente auf einem solchen Mikrochip oberflächenstrukturiert werden, um beispielsweise die Anheftung bestimmter Zellen oder die Platzierung von Hilfsstoffen oder Reagenzien zu ermöglichen oder zu optimieren. Weiterhin können auch einzelne Bereiche durch erfindungsgemäße Oberflächenstrukturen, die das Wachstum hemmen oder eine Anheftung verhindern, angepasst werden, um beispielsweise bestimmte Zellen von einer Reaktion auszuschließen. Die erfindungsgemäße Oberflächenstrukturierung bietet eine Vielzahl von Möglichkeiten, um einen Mikrochip für eine bestimmte Anwendung bzw. für bestimmte Reaktionen zu optimieren.

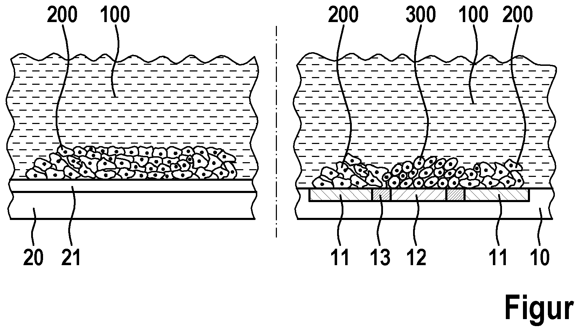

Weitere Vorteile und Merkmale der Erfindung ergeben sich aus der nachfolgenden Beschreibung von Ausführungsbeispielen in Verbindung mit der Zeichnung. Hierbei können die einzelnen Merkmale jeweils für sich oder in Kombination miteinander verwirklicht sein.

### Kurze Beschreibung der Zeichnung

Die Figur zeigt einen Ausschnitt des Bodens einer erfindungsgemäßen Zellkulturschale mit unterschiedlichen strukturierten Bereichen im Vergleich mit einer herkömmlichen Zellkulturschale.

### Beschreibung von Ausführungsbeispielen

Die **Figur** zeigt auf der rechten Seite eine erfindungsgemäße Zellkulturschale 10 bzw. einen Ausschnitt aus dem Boden der Zellkulturschale. Die Zellkulturschale 10 ist für eine Zellkultur von adhärenten Zellen vorgesehen, wobei in der Schale ein flüssiges Kulturmedium 100 enthalten ist, das die Zellen umspült. Die Zellkulturschale 10 bzw. der Boden der Zellkulturschale zeigt eine erfindungsgemäße Oberflächenstruktur 11, 12, 13, die in einzelne Bereiche untergliedert ist. Die einzelnen Strukturbereiche 11, 12 und 13 mittels elektromagnetischer Strahlung erzeugt worden und weisen jeweils eine Mikrostruktur auf, die von einer Nanostruktur überlagert ist. Durch entsprechende Einstellung der Prozessparameter sind unterschiedliche Strukturbereiche erzeugt worden, die sich insbesondere in der Größe und Form (Morphologie) der Mikrostrukturelemente und der Nanostrukturelemente unterscheiden. In diesem Beispiel ist der Strukturbereich 11 optimiert für die Proliferation und das Wachstum einer ersten Zellsorte 200. Der Strukturbereich 12 ist optimiert für die Anheftung und das Wachstum einer zweiten Zellsorte 300. Damit erlaubt die erfindungsgemäße Kulturschale 10 eine Co-Kultur der Zellen 200 und 300, auch wenn diese Zellen unterschiedliche Anforderungen an die Oberflächenbeschaffenheit des Substrates stellen. Weiterhin weist die erfindungsgemäße Kulturschale 10 einen Strukturbereich 13 auf, der für eine Nährstoffeinlagerung optimiert ist.

Hierdurch ist es möglich, dass die Zellen nicht nur über das Kulturmedium 100 mit Nährstoffen versorgt werden, sondern auch aus einem Depot auf der Gefäßoberfläche, also dem Strukturbereich 13. Dies ist besonders vorteilhaft, da mit zunehmendem Dickenwachstum der Zellschichten 200 und 300 die weiter unten liegenden Zellen nicht nur durch passiven Stofftransport versorgt werden. Bei einer herkömmlichen Zellkulturschale 20, die auf der linken Seite der Figur gezeigt ist, haben die Zellen bei zunehmender Schichtdicke der Zelllage nur in begrenzter Weise Zugang zu den erforderlichen Nährstoffen. Bei Verwendung der erfindungsgemäßen Kulturschale 10 kann daher ein größeres Dickenwachstum der Zellen 200 und 300 erreicht werden, wodurch größere Zelldichten realisierbar sind.

Auch der Boden einer herkömmlichen Zellkulturschale ist oftmals dahingehend an das Wachstum von adhärenten Zellen 200 angepasst, dass der Boden eine Beschichtung 21, beispielsweise mit Gelatine aufweist, die die Anheftung der Zellen 200 fördert. Mit zunehmendem Dickenwachstum werden jedoch insbesondere die unteren Zellschichten nur noch durch passiven Stofftransport mit Nährstoffen aus dem flüssigen Kulturmedium 100 versorgt, sodass das Dickenwachstum limitiert ist. Demgegenüber erlaubt die erfindungsgemäße Kulturschale 10 zum Einen eine verbesserte Nähstoffversorgung durch die Strukturbereiche 13 (Nährstoffeinlagerung) und zum Anderen eine Co-Kultur verschiedener Zellsorten 200, 300 durch die unterschiedlichen Strukturbereiche 11 und 12. Neben einer Einlagerung von Nährstoffen in den Bereichen 13 können beispielsweise auch zusätzlich oder alternativ hierzu Bereiche vorgesehen sein, die zur Bevorratung von bestimmten Wirkstoffen oder Wachstumsfaktoren, Interleukinen oder ähnlichem vorgesehen sind.

Eine derartige erfindungsgemäße Zellkulturvorrichtung kann mit Vorteil im Hinblick auf eine regenerative Medizin eingesetzt werden, um beispielsweise aus Knochenvorläuferzellen Knochenmaterial herzustellen. Hierfür ist das Einbringen von Wachstumsfaktoren und eine Co-Kultur mit Endothelzellen erforderlich. Übliche Zellkulturflaschen können zwar auf dem Flaschenboden eine für eine bestimmte Zellart optimierte Beschichtung aufweisen. Diese Beschichtung ist jedoch nicht für eine Co-Kultur geeignet, insbesondere wenn eine weitere Zellsorte andere Anforderungen an das Oberflächensubstrat stellt. In einer herkömmlichen Zellkulturflasche erfolgt die Nährstoffzufuhr über das Zellkulturmedium, wobei die Versorgung von unteren Zelllagen durch den passiven Stofftransport limitiert ist. Bei einem erfindungsgemäßen Zellkulturgefäß, beispielsweise einer Zellkulturschale oder einer Zellkulturflasche, kann das Gefäß für eine Co-Kultur verschiedenartiger Zellen vorbereitet und mit Nährstoffdepots oder Wirkstoffdepots in Anpassung an die jeweiligen Erfordernisse ausgestattet werden. Hierbei wird die Oberfläche entsprechend selektiv strukturiert und funktionalisiert, um die Bereiche für das Wachstum bestimmter Zellen zu optimieren.

Ein weiterer Anwendungsbereich der erfindungsgemäßen Oberflächenstrukturierung sind medizinische Implantate, beispielsweise Lang- oder Kurzzeitimplantate. In der Regel stehen Implantate nach deren Platzierung im Körper des Patienten mit körpereigenen Zellen in Kontakt. Je nach Anwendungsfall ist es erwünscht, dass körpereigene Zellen sich an dem Implantat anheften und anwachsen. Gegebenenfalls soll eine Anheftung von Zellen vermieden werden. In entsprechender Weise kann (können) erfindungsgemäß die Oberfläche oder Oberflächenbereiche eines Implantats strukturiert und angepasst werden. Beispielsweise kann bei einem Zahnimplantat der Schaftbereich, der im Knochenbett steckt, für das Anwachsen von Knochenzellen optimal strukturiert werden. Weiterhin kann beispielsweise knapp unterhalb des Bereichs, an dem das Zahnfleisch an dem Implantat anliegt, ein Depot für ein entzündungshemmendes Medikament oder entsprechende Wirkstoffe vorgesehen sein. Ein derartiges Depot kann ebenfalls durch eine erfindungsgemäße Oberflächenstrukturierung hergestellt werden.

Erfindungsgemäß ist es vorgesehen, die gewünschten Oberflächeneigenschaften direkt über eine Oberflächenmodifikation und nicht über eine Beschichtung, wie es herkömmlicherweise oftmals erfolgt, vorzunehmen. Eine Beschichtung birgt immer die Gefahr, dass die Beschichtung bei entsprechender Belastung oder infolge von Alterungseffekten teilweise oder vollständig abplatzt oder aufreißt. Erfindungsgemäß können die gewünschten Oberflächeneigenschaften durch Einstellung verschiedener Prozessparameter bei der Behandlung mit elektromagnetischer Bestrahlung, insbesondere mit einem Laserstrahl, erzeugt werden. Unterschiedliche Bereiche können ohne Weiteres durch entsprechende Bewegung des Laserstrahls und Wahl der Prozessparameter erzeugt werden, wobei diese Bereiche im Wesentlichen nur durch die Ausdehnung und Geometrie der Oberfläche der entsprechenden Vorrichtung selbst limitiert sind. Es ist kein Masken- oder anderes Abdeckverfahren wie bei üblichen Beschichtungen erforderlich, sodass die erfindungsgemäße Oberflächenstrukturierung sehr variabel und anpassbar ist.

Die oberflächenstrukturierten Vorrichtungen erlauben die Beeinflussung des Zell- und/oder Bakterienwachstums und/oder Gewebewachstums bei einer Vielzahl von Anwendungen, beispielsweise bei Zellkulturmaterialien, bei Lang- und Kurzzeitimplantaten oder auch bei Lab-on-a-Chip-Anwendungen. Diese Anwendungen bieten das Potential, verbesserte Bedingungen beispielsweise für den Bereich der regenerativen Medizin zu schaffen. Insbesondere kann mit der erfindungsgemäßen Oberflächenstrukturierung die Co-Kultur verschiedenartiger Zellen wesentlich verbessert und vereinfacht werden.

## Patentansprüche

1. Vorrichtung (10) für zellbiologische und/oder medizinische Anwendungen, wobeidie Vorrichtung wenigstens eine Oberfläche aufweist, die zumindest teilweise eine durch elektromagnetische Strahlung erzeugte Oberflächenstruktur (11, 12, 13) aufweist, die eine von einer Nanostruktur überlagerte Mikrostruktur aufweist, **dadurch gekennzeichnet, dass** die Oberfläche wenigstens zwei Bereiche (11, 12, 13) mit unterschiedlicher Oberflächenstrukturierung aufweist, wobei mindestens einer der Bereiche (11, 12, 13) für die Einlagerung von Substanzen, insbesondere Nährstoffen, Wachstumsstoffen oder Interleukinen, für eine Zell- oder Bakterienkultur optimiert ist

2. Vorrichtung nach Anspruch 1, **dadurch gekennzeichnet, dass** die Mikrostruktur Mikrostrukturelemente mit einem Durchmesser aus einem Größenbereich zwischen etwa 1 µm bis etwa 999 µm und/oder die Nanostruktur Nanostrukturelemente mit einem Durchmesser aus einem Größenbereich zwischen etwa 1 nm und etwa 999 nm aufweist.

3. Vorrichtung nach Anspruch 1 oder Anspruch 2, **dadurch gekennzeichnet, dass** die Oberflächenstruktur (11, 12, 13) durch Anpassung von Oberflächenstrukturelementen für das Wachstum vorgebbarer Zellsorten (200, 300) und/oder vorgebbarer Zelllinien optimiert ist.

4. Vorrichtung nach einem der Ansprüche 1 bis 3, **dadurch gekennzeichnet, dass** die Vorrichtung ein Gefäß (10) für die Zellkultur und/oder die Bakterienkultur und/oder die Gewebekultur ist.

5. Vorrichtung nach einem der Ansprüche 1 bis 4, **dadurch gekennzeichnet, dass** die Vorrichtung ein Gefäß (10) für die Co-Kultur verschiedener Zellen (200, 300) und/oder Bakterien ist.

6. Vorrichtung nach einem der Ansprüche 1 bis 3, **dadurch gekennzeichnet, dass** die Vorrichtung ein medizinisches Implantat ist.

7. Vorrichtung nach einem der Ansprüche 1 bis 3, **dadurch gekennzeichnet, dass** die Vorrichtung ein Mikrochip ist.

## Claims

1. Apparatus (10) for cytological and/or medical applications, wherein the apparatus has at least one surface that, at least in part, has a surface structure (11, 12, 13) produced by electromagnetic radiation, said surface structure having a microstructure on which a nanostructure is overlaid, **characterized in that** the surface has at least two regions (11, 12, 13) with different surface structuring, wherein at least one of the regions (11, 12, 13) is optimized for laying-in substances, in particular nutrients, growth substances or interleukins, for a cell or bacteria culture.

2. Apparatus according to Claim 1, **characterized in that** the microstructure has microstructure elements with a diameter from a size range of between approximately 1 µm to approximately 999 µm and/or the nanostructure has nanostructure elements with a diameter from a size range of between approximately 1 nm and approximately 999 nm.

3. Apparatus according to Claim 1 or Claim 2, **characterized in that** the surface structure (11, 12, 13) is optimized for the growth of predeterminable cell types (200, 300) and/or predeterminable cell lines by way of an adaptation of surface structure elements.

4. Apparatus according to any one of Claims 1 to 3, **characterized in that** the apparatus is a vessel (10) for the cell culture and/or the bacteria culture and/or the tissue culture.

5. Apparatus according to any one of Claims 1 to 4, **characterized in that** the apparatus is a vessel (10) for the co-culture of different cells (200, 300) and/or bacteria.

6. Apparatus according to any one of Claims 1 to 3, **characterized in that** the apparatus is a medical implant.

7. Apparatus according to any one of Claims 1 to 3, **characterized in that** the apparatus is a microchip.

## Revendications

1. Dispositif (10) pour des applications biologiques cellulaires et/ou médicales, le dispositif possédant au moins une surface qui présente au moins partiellement une structure de surface (11, 12, 13) générée par rayonnement électromagnétique, laquelle possède une microstructure à laquelle est superposée une nanostructure, **caractérisé en ce que** la surface présente au moins deux zones (11, 12, 13) ayant des structurations de surface différentes, au moins l'une des zones (11, 12, 13) étant optimisée pour le stockage de substances, notamment des nutriments, des substances de croissance ou des interleukines pour une culture de cellules ou de bactéries.

2. Dispositif selon la revendication 1, **caractérisé en ce que** la microstructure possède des éléments de microstructure ayant un diamètre dans une plage de grandeurs entre environ 1 µm et environ 999 µm et/ou la nanostructure possède des éléments de nanostructure ayant un diamètre dans une plage de grandeurs entre environ 1 nm et environ 999 nm.

3. Dispositif selon la revendication 1 ou 2, **caractérisé en ce que** la structure de surface (11, 12, 13) est optimisée par une adaptation des éléments de structure de surface pour la croissance de types cellulaires (200, 300) pouvant être prédéfinis et/ou de lignes cellulaires pouvant être prédéfinies.

4. Dispositif selon l'une des revendications 1 à 3, **caractérisé en ce que** le dispositif est un récipient (10) pour la culture de cellules et/ou la culture de bactéries et/ou la culture de tissus.

5. Dispositif selon l'une des revendications 1 à 4, **caractérisé en ce que** le dispositif est un récipient (10) pour la coculture de différentes cellules (200, 300) et/ou bactéries.

6. Dispositif selon l'une des revendications 1 à 3, **caractérisé en ce que** le dispositif est un implant médical.

7. Dispositif selon l'une des revendications 1 à 3, **caractérisé en ce que** le dispositif est un microcircuit.
